# EUROPÄISCHE PATENTANMELDUNG

(11) **EP 0 615 974 A1**
(43) Veröffentlichungstag der Anmeldung: **21.09.1994**
(21) Anmeldenummer: 94103872.1
(22) Anmeldetag: 14.03.1994
(51) Int. Cl.: C07H 15/04

(54) **Verfahren zur Herstellung von Alkylglycosiden**

(30) Priorität: 19.03.1993 EP 93104577
(71) Anmelder: Akzo Nobel N.V., NL-6824 BM Arnhem (NL)
(72) Erfinder: Bergfeld, Manfred Josef, Dr., D-63906 Erlenbach (DE); Seifert, Jürgen, Dr., D-63868 Grosswallstadt (DE)
(74) Vertreter: Fett, Günter

(57) **Zusammenfassung**

Es wird ein Verfahren zur Herstellung von Alkylglycosiden durch Umsetzung von Monosacchariden mit Fettalkoholen in Gegenwart eines sauren Katalysators beschrieben, das dadurch gekennzeichnet, daß man als Katalysator perfluorierte Sulfonsäuren verwendet.

## Beschreibung

Die Erfindung betrifft ein Verfahren zur Herstellung von Alkylglycosiden durch direkte Synthese, ausgehend von Fettalkoholen und Sacchariden unter Verwendung von sauren Katalysatoren. Unter Alkylglycosiden sind im Rahmen der Erfindung Verbindungen zu verstehen, bei denen Alkylreste acetalisch an monomere und/oder oligomere Zuckerreste gebunden sind.

Unter Alkylreste werden Reste verstanden, die sich von monofunktionellen Alkoholen ableiten, die linear oder verzweigt sein können und bevorzugt 8 bis 20 Kohlenstoffatome besitzen. Besonders geeignete Alkylreste sind solche, die sich von Alkoholen ableiten, die aus Naturstoffen wie Fetten und Ölen gewonnen werden und meistens ein Gemisch darstellen, z.B. C₁₂/C₁₄-Ketten besitzen und auch ungesättigte Reste aufweisen können.

Unter Fettalkoholen im Rahmen der Erfindung sind somit sowohl aus Naturprodukten gewonnene Alkohole als auch synthetisch hergestellte Alkanole zu verstehen.

Es ist bekannt, Alkylglycoside aus Fettalkoholen und Sacchariden unter Verwendung eines sauren Katalysators herzustellen. Es werden in der Literatur zahlreiche Methoden beschrieben, bei denen zunächst ein Glycosid aus Sacchariden durch Umsetzung eines niedermolekularen Alkohols wie Methanol, Äthanol u.dgl. gewonnen wird, das sodann durch Umacetalisierung mit einem höhermolekularen Alkohol in das gewünschte Alkylglycosid umgewandelt wird. Für diesen Reaktionstyp sind zahlreiche Katalysatoren empfohlen worden.

Es ist ferner bekannt, aus Sacchariden direkt durch Umsetzung mit einem höhermolekularen Alkohol, d.h. Alkohole mit 8 bis beispielsweise 22 Kohlenstoffatomen zu synthetisieren. Auch für diese Reaktionsweise sind in der Literatur bereits zahlreiche saure Katalysatoren erwähnt.

So werden, wie z.B. der europäischen Patentanmeldung 0 132 043 zu entnehmen ist, anorganische Säuren wie Salzsäure, Schwefelsäure, Phosphorsäure empfohlen; auch Paratoluolsulfonsäure, Bortrifluorid werden zu diesem Zweck angegeben. Nach der Lehre der europäischen Patentanmeldung 0 132 043 werden anionische, oberflächenaktive Mittel wie Alkylsulfate, Alkylbenzolsulfonate und Alkylsulfonate als Katalysatoren für die Herstellung von Alkylglycosiden eingesetzt.

In Recl. Trav. Chim. Pays-Bas 110, 023-024 (1991) wird die Umsetzung von Monosacchariden mit 4-Pentenol in Dimethylsulfoxid als Lösungsmittel unter Verwendung von Trifluormethansulfonsäure beschrieben. Dabei handelt es sich um ein Glycosid mit einem niedermolekularen Alkohol, das als Waschrohstoff nicht geeignet ist. Darüberhinaus ist die Ausbeute nach einer Reaktionszeit von 2 Tagen bei 90°C, wenn man Glucose verwendet, nur 56%.

In J. Carbohydrate Chemistry, 8(1) 263-269 (1988) wird die Umsetzung von Zuckern mit Allylalkohol beschrieben, wobei als Katalysator Trifluormethansulfonsäure eingesetzt wird. Dieses Glycosid aus einem niedermolekularen Alkohol wird sodann mit Benzylalkohol umacetalisiert. Über eine brauchbare Direktsynthese von waschaktiven Alkylglycosiden ist dieser Schrift nichts zu entnehmen.

Obwohl bereits die verschiedensten Verfahren für die Herstellung von Alkylglycosiden, sei es auf direktem oder indirektem Weg unter der Verwendung von den verschiedensten Katalysatoren bekannt sind, besteht noch ein Bedürfnis nach verbesserten Verfahren der Direktsynthese.

Aufgabe der Erfindung ist es deshalb, ein Verfahren zur Direktsynthese von Alkylglycosiden ausgehend aus Fettalkoholen mit 8 bis 22 Kohlenstoffatomen und Sacchariden, insbesondere Monosacchariden unter der Verwendung von sauren Katalysatoren zur Verfügung zu stellen, das wirtschaftlich durchzuführen ist, das für gegebene Reaktionsbedingungen schnellere Reaktionszeiten ermöglicht, das zu einem Produkt mit guten Eigenschaften führt und das einfach handhabbar ist und zu reproduzierbaren Ergebnissen führt.

Aufgabe der Erfindung ist es ferner, ein Verfahren zur Verfügung zu stellen, das mit geringeren Mengen an Katalysator auskommt, als die bisher bekannten. Aufgabe der Erfindung ist es ferner, ein Verfahren zur Verfügung zu stellen, das auch bei niedrigeren Temperaturen bei wirtschaftlich akzeptierbaren Reaktionszeiten zu zufriedenstellenden Ausbeuten führt.

Diese Aufgabe wird gelöst durch ein Verfahren zur Herstellung von Alkylglycosiden durch Umsetzung von Monosacchariden mit Fettalkoholen in Gegenwart eines sauren Katalysators, dadurch gekennzeichnet, daß man Monosaccharide und Fettalkohole mit einer Kohlenstoffzahl von 8 bis 22 in Gegenwart von perfluorierten Sulfonsäuren als Katalysator umsetzt. Sehr geeignet sind perfluorierte Alkansulfonsäuren mit mindestens 2 Kohlenstoffatomen wie z.B. Perfluoroctansulfonsäure, Perfluorbutansulfonsäure, Perfluorpropansulfonsäure, Perfluorethansulfonsäure sowie deren Gemische. Sehr bewährt haben sich auch perfluorierte Cycloalkansulfonsäuren wie z.B. 1,4-Perfluorethylcyclohexansulfonsäure. In einer weiteren vorteilhaften Ausführungsform des erfindungsgemäßen Verfahrens werden perfluorierte Sulfocarbonsäuren und/oder deren Ester, z.B. Perfluorsulfoessigsäure verwendet. Vorzugsweise wird als Monosaccharid Glucose verwendet. Es ist vorteilhaft, 0,5 bis 15 mMol Katalysator pro Mol Monosaccharid einzusetzen. Die Menge des Katalysators kann natürlich in weiteren Bereichen variiert werden, sowohl nach oben als auch nach unten und z.B. 0.1 mMol oder 20 mMol pro Mol Monosaccharid betragen.

In einer besonders vorteilhaften Ausführungsform wird die Umsetzung von Monosaccharid mit Fettalkohol kontinuierlich durchgeführt.

Es war besonders überraschend, daß das erfindungsgemäße Verfahren vielfach in erheblich kürzeren Zeiten zu einem zufriedenstellenden Umsatz des Fettalkohols mit dem Monosaccharid führt. Aufgrund der hohen Geschwindigkeiten ist es möglich, die Umsetzung bei niedrigeren Temperaturen durchzuführen, als das der Fall ist, wenn man übliche für derartige Umsetzungen bekannte Katalysatoren verwendet. Außerdem ist es möglich mit erheblich geringeren Mengen an Katalysator auszukommen. Der Katalysator läßt sich im übrigen, sofern dies überhaupt erwünscht oder erforderlich ist, in hohen Ausbeuten wieder regenerieren und erneut bei der Synthese einsetzen. Aus diesem Grund arbeitet das Verfahren sehr umweltfreundlich.

Das Verfahren läßt sich in besonders vorteilhafter Weise bei hohen Ausbeuten kontinuierlich durchführen. Es ist nicht erforderlich, zunächst Glycoside mit niedrigeren Alkoholen herzustellen und anschließend umzuacetalisieren.

Da die im Endprodukt verbleibende Menge an Katalysator vernachlässigbar gering ist, kann er meistens auch im Endprodukt verbleiben.

Vor allem ist die Aktivität bei sehr feinteiliger Glucose besonders hoch, verglichen mit üblichen Katalysatoren.

Es war ferner überraschend, daß das erfindungsgemäße Verfahren Alkylglycoside liefert, die sich hinsichtlich verschiedener Eigenschaften von nach üblichen Verfahren hergestellten Produkten unterscheiden. So ist es z.B. möglich, insbesondere bei kontinuierlicher Fahrweise, Alkylglycoside zu erhalten, die bei sonst gleicher Waschkraft im wässrigen System eine erheblich niedrigere Viskosität aufweisen. Dies bringt mit sich, daß man einmal wässrige Systeme mit erheblich höheren Konzentrationen an Alkylglycosid einstellen kann, so können Systeme mit bis zu 75% Alkylglycosid aufgemacht werden. Ein weiterer Vorteil des erfindungsgemäßen Verfahrens ist es, daß es die Herstellung von Produkten erlaubt, die auch noch in höheren Konzentrationen als wässrige Aufmachungen pumpbar sind. Übliche Produkte sind bei höheren Konzentrationen wie beispielsweise 50% oder darüber allenfalls als Pasten aufzumachen, die, sollten sie gepumpt werden, erwärmt werden müssen, was mit erheblichen Nachteilen verbunden ist. Die erfindungsgemäß erhaltenen Produkte hingegen lassen sich noch bis in hohe Konzentrationen bei Zimmertemperatur pumpen. Die Grenzflächenspannungswerte und die Solubilisierung der erfindungsgemäß hergestellten Produkte sind hervorragend. Die Schaumwerte wie Schaumhöhe, Schaumstabilität u.dgl. sind gut.

Es ist ferner ein großer Vorteil, daß man auch sirupöse Glucose umsetzen kann. Sirupöse Glucose ist ein wohlfeiles Produkt, das man z.B. durch saure oder enzymatische Hydrolyse von Stärke erhält. Sirupöse Glucosen können auch aus Saccharose gewonnen werden, wie man sie beispielsweise aus Zuckerrüben erhält. Sirupöse Glucose enthält neben Glucose und Wasser auch noch Oligomere und zu einem geringen Teil Polysaccharide.

Die Erfindung wird durch folgende Beispiele näher erläutert:

### Allgemeine Vorgehensweise und Beschreibung der verwendeten Apparatur

Als Reaktor wurde ein handelsüblicher 1 L Büchi-Glasreaktor mit Doppelmantelbeheizung, Intermig-Rührer, Bodenablaß und Destillationsaufsatz verwendet.

Die folgenden Beispiele wurden im batch-Betrieb gefahren, d.h. die gesamte Glucose (Glc) und der gesamte Fettalkohol (FA) wurden im Reaktor vorgelegt und das Gemisch unter Rühren und Evakuieren auf 20 mbar auf Solltemperatur gebracht.

Danach wurde der Katalysator zugegeben und dieser Zeitpunkt als Reaktionsbeginn gewertet.

Das Reaktionswasser wurde kontinuierlich als Dampf abgezogen und in ein graduiertes Gefäß niedergeschlagen. Die Wasserentstehungsrate wurde als Stoffänderungsgeschwindigkeit der Glc gewertet (pro Mol umgesetztes Glc wird 1 Mol Wasser frei).

Die notwendige Zeit für 99% Glc-Umsatz wurde als Reaktionszeit gewertet und für die angestellten Vergleiche verwendet.

### Beispiel 1

90.5 g Glucose wasserfrei (Cerestar) mit einem mittleren Konrdurchmesser von 5 µm wurden zusammen mit 412.0 g Fettalkohol (Nafol 1214 von Condea enthaltend ca. 54% Lauryl- und ca. 44% Myristylalkohol) im Reaktor vorgelegt und nach Evakuieren auf 20 mbar unter Rühren auf die Reaktionstemperatur von 110°C gebracht. Nach Erreichen von 110°C wurden 5 mmol Perfluoroctansulfonsäure pro mol Glucose zugegeben. Nach 60 min Reaktionszeit bei 110°C ± 1°C waren 497 mmol Reaktionswasser (= 8.95 g) aus dem Reaktor abgezogen, was 99.0% Glucose-Umsatz entspricht.

Das Reaktionsprodukt war klar und weiß.

Nach der Neutralisation des Katalysators mit NaOH und einer FA-Abtrennung auf 1.5 Gew.% wurden folgende Analysen enthalten:

| | |
|---|---|
| freie Glucose: | < 1 Gew.% |
| Monoglucosid (C₁₂ + C₁₄): | 53.0 Gew.% |

### Beispiel 2

Dieses Beispiel wurde analog zu Beispiel 1 gefahren mit der Ausnahme, daß als Katalysator ein Gemisch von Perfluorethan-, Perfluorpropan- und Perfluorbutansulfonsäure mit einer mittleren Molmasse von 253.2 g/mol verwendet wurde. Nach ebenfalls 60 min Reaktionszeit waren 99% der eingesetzten Glucose umgesetzt.

Das weiße, klare Reaktionsprodukt zeigte nach einer FA-Abtrennung auf 1.8 Gew.% folgende Analysenwerte:

| | |
|---|---|
| freie Glucose: | < 1 Gew.% |
| Monoglucosid (C₁₂ + C₁₄): | 53.2 Gew.% |

### Vergleichsbeispiel 3

Dieses Beispiel wurde analog zu Beispiel 1 gefahren, mit der Ausnahme, daß ein Katalysator nach dem Stand der Technik (p-Toluolsulfonsäure) verwendet wurde.

Für 99% Glucose-Umsatz waren hierbei jedoch 180 min, also die dreifache Zeit im Vergleich zu den erfindungsgemäßen Katalysatoren notwendig.

Das Rohprodukt war leicht trübe und von beiger Farbe; es wies nach einer Abtrennung des überschüssigen FA auf 1.4% folgende Werte auf:

| | |
|---|---|
| freie Glucose: | < 1 Gew.% |
| Monoglucosid (C₁₂ + C₁₄): | 49.8 Gew.% |

## Patentansprüche

1. Verfahren zur Herstellung von Alkylglycosiden durch Umsetzung von Monosacchariden mit Fettalkoholen in Gegenwart eines sauren Katalysators, dadurch gekennzeichnet, daß man als Katalysator perfluorierte Sulfonsäuren verwendet.

2. Verfahren nach Anspruch 1, dadurch gekennzeichnet, daß man perfluorierte Alkansulfonsäuren mit mindestens 2 Kohlenstoffatomen oder deren Gemische verwendet.

3. Verfahren nach Anspruch 1, dadurch gekennzeichnet, daß man perfluorierte Cycloalkansulfonsäuren verwendet.

4. Verfahren nach einem der Ansprüche 1 bis 3, dadurch gekennzeichnet, daß man perfluorierte Sulfocarbonsäuren und/oder deren Ester verwendet.

5. Verfahren nach einem oder mehreren der Ansprüche 1 - 4, dadurch gekennzeichnet, daß man 0,5 bis 15 mMol Katalysator Mol Monosaccharid verwendet.

6. Verfahren nach einem oder mehreren der Ansprüche 1 bis 5, dadurch gekennzeichnet, daß man als Monosaccharid Glucose verwendet.

7. Verfahren nach Anspruch 6, dadurch gekennzeichnet, daß man feingemahlene Glucose verwendet.

8. Verfahren nach Anspruch 7, dadurch gekennzeichnet, daß man Glucose mit einem mittleren Teilchendurchmesser von 3 bis 4 µm verwendet.

9. Verfahren nach einem oder mehreren der Ansprüche 1, 2 und 5 - 8, dadurch gekennzeichnet, daß man als perfluorierte Sulfonsäure Perfluoroctansulfonsäure verwendet.

10. Verfahren nach einem oder mehreren der Ansprüche 1, 2 und 5 - 8, dadurch gekennzeichnet, daß man als perfluorierte Sulfonsäure Perfluorbutansulfonsäure verwendet.

11. Verfahren nach einem oder mehreren der Ansprüche 1, 2 und 5 bis 8, dadurch gekennzeichnet, daß man als Katalysator Gemische von Perfluorethan-, Perfluorpropan- und Perfluorbutansulfonsäure verwendet.

12. Verfahren nach einem oder mehreren der Ansprüche 1 bis 11, dadurch gekennzeichnet, daß man die Umsetzung kontinuierlich durchführt.
